Europäisches Patentamt

European Patent Office

Office européen des brevets

(19)

(11) **EP 1 566 639 A1**

(12) **EUROPEAN PATENT APPLICATION**
published in accordance with Art. 158(3) EPC

(43) Date of publication:
**24.08.2005 Bulletin 2005/34**

(51) Int Cl.⁷: **G01N 33/547**, G01N 33/543,
G01N 33/553

(21) Application number: **03810653.0**

(22) Date of filing: **07.11.2003**

(86) International application number:
**PCT/JP2003/014201**

(87) International publication number:
**WO 2004/042401 (21.05.2004 Gazette 2004/21)**

(84) Designated Contracting States:
**AT BE BG CH CY CZ DE DK EE ES FI FR GB GR
HU IE IT LI LU MC NL PT RO SE SI SK TR**
Designated Extension States:
**AL LT LV MK**

(30) Priority: **08.11.2002 JP 2002326193**

(71) Applicants:
• **Takahashi, Hiroshi
Saitama 339-0045 (JP)**
• **Hanada, Shuichi
Kagoshima 891-0175 (JP)**

(72) Inventors:
• **TAKAHASHI, Hiroshi
Iwatsuki-shi, Saitama 339-0045 (JP)**
• **HANADA, Shuichi
Kagoshima-shi, Kagoshima 891-0175 (JP)**
• **MITSUNAGA, Makoto
Bunkyo-ku, Tokyo 112-0011 (JP)**

(74) Representative: **Wytenburg, Wilhelmus Johannes
Mewburn Ellis LLP,
York House,
23 Kingsway
London WC2B 6HP (GB)**

(54) **METHOD OF EXAMINING CANCER CELLS AND REAGENT THEREFOR**

(57) A method for examining cancer cells by which examination of cancer cells may be carried out simply and efficiently without using an expensive apparatus, and a reagent therefor are disclosed. In the method for examining cancer cells according to the present invention, cancer cells separated from the body, which cells express SF-25 antigen on their surfaces, are bound to magnetic beads utilizing antigen-antibody reaction between the cancer cells and an anti-SF-25 antibody or antigen-binding fragment thereof, and then the magnetic beads are collected by magnetic force, and the cancer cells bound to the magnetic beads are examined.

SF-25-positive Cells in Peripheral Blood Mononuclear Cells in Each Clinical Form

Fig. 1

**Description**

Technical Field

The present invention relates to a method for examining cancer cells and reagent therefor.

Background Art

Collection of cells expressing a characteristic antigen on the cell surface is hitherto carried out by utilizing the characteristic antigen as a marker. The most commonly employed method today is the method in which a fluorescence-labeled antibody is reacted with the antigen on the cells, and the cells are separated by flow cytometry utilizing the fluorescent label of the bound antibody (for example, section of "cell sorter" in Nikkei Bio Latest Dictionary, 5th Edition). This apparatus is called cell sorter. However, the cell sorter is an expensive apparatus which costs as high as several tens million Yen. Although the apparatus is convenient, the purity of the collected cell population is not necessarily high satisfactorily.

A method for separating and collecting cells expressing a marker antigen on the cell surfaces using magnetic beads on which an antibody to the marker antigen expressed on the cell surfaces is also known (e.g., Japanese Laid-open PCT Application Nos. 8-510390 and 2001-522806). However, it is not known to search the nucleic acid of the cell population collected by using the magnetic beads and to use the cells for examination of cancer.

Disclosure of the Invention

An object of the present invention is to provide a method for examining cancer cells by which examination of cancer cells may be carried out simply and efficiently without using an expensive apparatus, and a reagent therefor.

The present inventors intensively studied to discover that diagnosis of cancer may be attained by collecting cancer cells expressing SF-25 antigen by binding the cancer cells to magnetic beads by utilizing an anti-SF-25 antibody, and examining the cells bound to the magnetic beads, thereby completing the present invention.

That is, the present invention provides a method for examining cancer cells, comprising binding cancer cells separated from the body, which cells express SF-25 antigen on their surfaces, to magnetic beads utilizing antigen-antibody reaction between said cancer cells and an anti-SF-25 antibody or antigen-binding fragment thereof, then collecting said magnetic beads by magnetic force, and examining said cancer cells bound to said magnetic beads. The present invention also provides a reagent for examination of cancer cells for carrying out the method according to the present invention, comprising magnetic beads on which an anti-SF-25 antibody or antigen-binding fragment thereof is immobilized.

By the method of the present invention, examination of cancer cells may be attained simply and efficiently without using an expensive apparatus such as cell sorter. Collection of magnetic beads may be carried out by an apparatus using magnet or manually using a magnet, so that it can be carried out much more inexpensively than the method using a cell sorter. Further, by the method according to the present invention using the magnetic beads, the purity of the collected cells is higher than that attained by the method using a cell sorter, that is, SF-25 antigen-expressing cells alone may be accurately collected, so that the subsequent examination may be carried out efficiently and accurately.

Brief Description of the Drawings

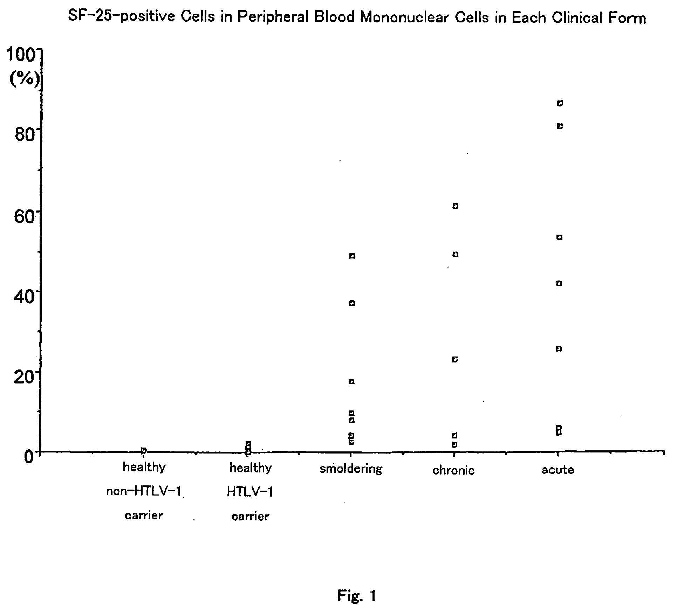

Fig. 1 shows the ratio of SF-25 antigen-positive mononuclear cells in the total mononuclear cells, which was measured by flow cytometry using an anti-SF-25 monoclonal antibody, using the mononuclear cells in peripheral blood from acute ATL patients, chronic ATL patients, smoldering ATL patients, healthy ATL carriers and healthy individuals (not infected with HTLV-1) as samples, in an Example of the present invention.

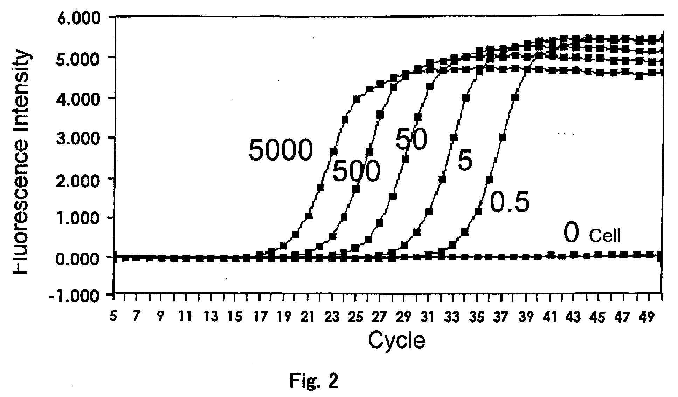

Fig. 2 shows the relationship between the number of cycles and the fluorescence intensity when pX gene at various concentrations was amplified in an Example of the present invention.

Best Mode for Carrying Out the Invention

As mentioned above, according to the method for examining cancer cells of the present invention, the cancer cells separated from the body, which express SF-25 antigen on their surfaces, are bound to magnetic beads utilizing antigen-antibody reaction between the cancer cells and the anti-SF-25 antibody or antigen-binding fragment thereof.

SF-25 is a glycoprotcin antigen having a molecular weight of about 125 kDa found in 1987 (WO89/05307; European Patent No. 0 397 700; U.S. Patent No. 5,212,085; Takahashi H, Wilson B, Ozturk M, Mottc P, Strauss W, Isselbacher KJ and Wands JR. In vivo localization of colon adenocarcinoma by monoclonal antibody binding to a highly expressed cell surface antigen. *Cancer Research* 1988; 48: 6573-6579., Wilson B, Ozturk M, Takahashi H, Motte P, Kew M, Isselbacher KJ and Wands JR. Cell surface changes associated with transformation of human hepatocytes to the malignant phenotype. *Proc. Natl. Acad. Sci. USA* 1988; 85: 3140-3144., Takahashi H, Carlson R, Ozturk M, Sun S, Motte P, Strauss W, Isselbacher KJ, Wands JR and Shouval D. Radioimmunolocalization of hepatic and pulmonary metastasis of human colon adenocarcinoma. *Gastroenterology* 1989; 96: 1317-1329., Hurwitz E, Stancovski I, Wilcheck M, Shouval D, Takahashi H, Wands JR, Sela M. A conjugate of 5-Fluorourodine-poly(L-lysinc) and an antibody reactive with human colon carcinoma. *Bioconjugate Chemistry* 1990; 1: 285-290., Wands JR, Takahashi H. Studies on cell surface changes associated with transformation of human hepatocytes to the malignant phenotype and their role as potential immunotargeting sites. In *Frontiers of Mucosal Immunology.* Volume 2. Eds by Tsuchiya M. 1991 pp. 295-298. Hurwitz E, Adler R, Shouval D, Takahashi H, Wands JR, Sela M. Immunotargeting of daunomycin to localized and metastatic human colon adenocarcinoma in athymic mice. *Cancer Immunology Immunotherapy* 1992; 35: 186-192., Takahashi H, Nakada T, Puisieux I. Inhibition of human colon cancer growth by antibody-directed human LAK cells in SCID mice. *Science* 1993; 259: 1460-1463., Takahashi H, Nakada T, Nakaki M, Wands JR. Inhibition of hepatic metastases of human colon cancer in nude mice by a chimeric SF-25 monoclonal antibody. G*astroenterology* 1995; 108: 172-182). SF-25 antigen is known to express on human colon cancer cell lines (e.g., LS 180 (ATCC No. CL0187), COLO 320 (ATCC No. CCL-220.1), WiDr (ATCC No. CCL-218), Caco-2 (HTB-37) and on human liver cancer cell lines (e.g., FOCUS (Lun H. et al., in Vitro 20; 493-504 (1984)). Anti-SF-25 monoclonal antibodies are also known (WO89/05307, European Patent No. 0 397 700 and U.S. Patent No. 5,212,085), and a hybridoma producing an anti-SF-25 monoclonal antibody is deposited with ATCC (ATCC No. HB9599).

The anti-SF-25 antibody may preferably be a monoclonal antibody. As mentioned above, anti-SF-25 monoclonal antibodies are known and one of them has been deposited. The monoclonal antibody produced by the deposited hybridoma ATCC No. HB9599 was prepared by immunizing mice with the above-described human liver cancer cell line FOCUS, preparing hybridomas producing monoclonal antibodies, and selecting a monoclonal antibody which undergoes antigen-antibody reaction with the above-described various human colon cancer cell lines. In the method of the present invention, the deposited anti-SF-25 monoclonal antibody may be employed, and other monoclonal antibodies prepared by the similar method may also be employed. As concretely described in WO89/05307, European Patent No. 0 397 700 and U.S. Patent No. 5,212,085, describing ATCC No. HB9599, since a number of hybridomas other than ATCC No. HB9599 were obtained in one preparation process, an anti-SF-25 monoclonal antibody may be easily prepared by the known method. Therefore, the monoclonal antibody used in the method of the present invention is not restricted to that produced by the deposited hybridoma. Further, not only the entire antibody, but also fragments thereof having an ability to bind with the antigen, such as Fab fragment and F(ab')$_2$ fragment, may also be employed.

Magnetic beads are particles prepared by giving magnetism to latex particles or polystyrene particles by, for example, blending ferrite. Magnetic beads carrying an antigen or antibody are well-known in the field of immunoassay and are commercially available. As the magnetic beads used in the present invention, those which do not adsorb the cells, by non-specific interaction, other than the target cells bound through SF-25 antibody are desired, and DYNABEADS (trademark, Dynal Biotech) and the like may preferably be employed.

The method for binding the cancer cells separated from the body, which cells express on their surfaces the SF-25 antigen to the magnetic beads utilizing the antigen-antibody reaction between the cancer cells and the anti-SF-25 antibody or antigen-binding fragment thereof include direct method and indirect method, and either of them may be employed. The direct method is the method in which an anti-SF-25 antibody or the antigen-binding fragment thereof is immobilized on the magnetic beads, and the cancer cells are directly bound to the magnetic beads by the antigen-antibody reaction between the anti-SF-25 antibody or antigen-binding fragment thereof and the cancer cells. The direct method has an advantage that it can be carried out quickly and simply because the antigen-antibody reaction is carried out only once.

The method *per se* for immobilizing an antibody or the antigen-binding fragment thereof to the magnetic beads is well-known. For example, magnetic beads may be applied to a solution of the antibody or its antigen-binding fragment so as to physically adsorb the antigen or the antigen-binding fragment on the magnetic beads. In this case, the concentration of the antibody or the antigen-binding fragment thereof in the mixture may be, although not restricted, usually about 0.001 to 1% by weight, and the concentration of the magnetic beads may be, although not restricted, usually about 0.1 to 50% by weight. Although the conditions for the physical adsorption are not restricted, it may be usually carried out by incubating the mixture at 4°C to 45°C for about 0.5 to 48 hours. The method for immobilization of the antibody or the antigen-binding fragment thereof onto the magnetic beads is not restricted to physical adsorption, and other known methods, for example, covalently bonding the antibody or the antigen-binding fragment thereof using functional groups such as amino groups or carboxyl groups bound to the magnetic beads may also be employed.

On the other hand, the indirect method is a method in which the cancer cells and a labeled or non-labeled anti-SF-25 antibody or antigen-binding fragment thereof are subjected to antigen-antibody reaction to bind the antibody or antigen-binding fragment thereof to the cells, and subsequently or simultaneously, the cells are bound to the magnetic beads through the antibody or antigen-binding fragment thereof by specific reaction between the antibody or antigen-binding fragment thereof or the label attached thereto and the magnetic beads. In cases where a non-labeled antibody or antigen-binding fragment thereof is used, an antibody or antigen-binding fragment thereof which undergoes antigen-antibody reaction with the non-labeled antibody or antigen-binding fragment thereof is immobilized on the magnetic beads. For example, in cases where the anti-SF-25 antibody which is to be reacted with the cancer cells is a mouse IgG, an anti-mousc IgG antibody may be immobilized on the magnetic beads. In cases where a labeled antibody or antigen-binding fragment thereof is bound to the cancer cells, an antibody or antigen-binding fragment thereof or a substance which specifically binds to the label is immobilized on the magnetic beads. Therefore, as the label, any substance which has antigenecity and which does not hinder the antigen-antibody reaction between the cancer cells and the anti-SF-25 antibody or antigen-binding fragment thereof may be employed. For example, in Examples below, fluorescein isothiocyanate (FITC) as a label, which is well-known as a fluorescent label, is bound to an anti-SF-25 monoclonal antibody, and the FITC is bound to anti-FITC antibody-immobilized magnetic beads. Alternatively, biotin may be used as the label, and the biotin may be bound to the magnetic beads on which avidin or an avidin derivative such as streptoavidin is immobilized. Thus, as the label, any substance may be employed, which can specifically bind to another substance, and which does not hinder the antigen-antibody reaction between the cancer cells and the anli-SF-25 antibody or antigen-binding fragment thereof. Anti-mouse IgG antibody-immobilized magnetic beads, anti-FITC antibody-immobilized magnetic beads, streptoavidin-immobilized magnetic beads and the like are widely used in the field of immunoassay, and are commercially available because their versatility is high. In the method of the present invention, these commercially available magnetic beads may preferably be employed.

The cancer cells which may be examined by the method of the present invention are any cancer cells expressing the SF-25 antigen on their surfaces, and examples of the cancer cells include leukemia cells, colon cancer cells, small intestinal cancer cells, gastric cancer cells, esophagus cancer cells, bile duct cancer cells, gallbladder cancer cells, thyroid cancer cells, parathyroid cancer cells, prostate cancer cells, uterine cancer cells, ovarian cancer cells, choriocarcinoma cells, orchioncus cells, bladder cancer cells, renal cancer cells, adrenal cancer cells, brain tumor cells, melanoma cells, skin cancer cells, lung cancer cells, breast cancer cells, pancreatic cancer cells and liver cancer cells. Examples of the leukemia cells include transformed lymphocytes and mononuclear cells. In Example 1 below, adult T cell leukemia (ATL) accompanying transformed lymphocytes is examined, but it was hitherto not known that SF-25 antigen is expressed on transformed lymphocytes.

In a preferred mode of the present invention, blood or cells separated from the blood are used as the tcst sample. Among the various cancer cells described above, leukemia cells are contained in the blood, so that it is natural that the blood or the cells separated from the blood may be tested by the method of the present invention. Further, since solid cancer cells, peeled off from the tissues, such as colon cancer cells, gastric cancer cells, lung cancer cells, breast cancer cells, pancreatic cancer cells and liver cancer cells, are also contained in the blood, cerebrospinal fluid, bone marrow, pleural effusion, ascites, pancreatic juice, duodenal juice, bile, feces or urine, examination of these solid cancer cells may be attained by the method of the present invention using blood or the like. Although biopsy is necessary for obtaining solid cancer cells from an organ tissue, by the method of the present invention, blood that can be much more easily and safely obtained than organ tissues may be used as the tcst sample, which is advantageous.

In the direct method, although the conditions for the antigen-antibody reaction between the magnetic beads on which the antibody or antigen-binding fragment thereof is immobilized and the cancer cells are not restricted, in cases where the test cells (a mixture of normal cells and cancer cells) have already been separated, the antigen-antibody reaction may be carried out by bringing the magnetic beads into contact with the cancer cells, for example, at about 4°C to 45°C for about 0.5 to 24 hours. The population density in the mixture used herein is not restricted, and usually about 1 cell/ml to $10^6$ cells/ml, and the concentration of the magnetic beads may be, although not restricted, usually about 0.1 to 10% by weight. In cases where blood is made to contact with magnetic beads, it may be carried out at a temperature between about 4 and 45°C for about 0.5 to 24 hours. In this case, the concentration of the magnetic beads in the mixture may be as described above. In the case of indirect method, the concentration of the anti-SF-25 antibody or antigen-binding fragment thereof which is subjected to the antigen-antibody reaction with the cancer cells may be, although not restricted, usually about 0.001 to 1% by weight. As for the conditions for the antigen-antibody reaction, either the reaction between the cancer cells and the anti-SF-25 antibody or antigen-binding fragment thereof or the reaction between the produced antigen-antibody complex and the antibody or antigen-binding fragment thereof on the magnetic beads may be carried out under the conditions similar to those employed in the above-described direct method. In the indirect method, the antigen-antibody reaction between the cancer cells and the anti-SF-25 antibody or antigen-binding fragment thereof may be carried out firstly, and then the produced antigen-antibody complex may be reacted with the magnetic beads. Alternatively, the cancer cells, the anti-SF-25 antibody or antigen-binding fragment thereof and the magnetic beads may be made to co-exist, and the above-described two rcactions may be carried out in parallel. In cases where a specifically binding substance such as biotin is used as the label, the reaction may be carried out under the conditions well-known for the respective label.

Thereafter, magnetic beads are collected by magnetic force. As mentioned above, since immunoassay using magnetic beads is well-known, and since the apparatuses for collecting magnetic beads by magnetic force are commercially available, collection of the magnetic beads may be carried out easily using a commercially available apparatus. Alternatively, the collection may be attained manually by simply using a magnet.

By the above-described steps, the cells expressing SF-25 antigen on their surfaces are bound to the magnetic beads. Examination of the cancer cells bound to the magnetic beads is then carried out. It is preferred to conduct a washing step in which the magnetic beads are washed with a buffer solution and the magnetic beads are collected again by magnetic force, before the examination. The examination *per se* of the cells may be carried out by a method known for the respective cancer cells. For example, an examination by which the cells can be identified as cancer cells, such as examination of nucleic acid, pathological examination or biochemical examination is carried out. In Examples 1 and 3 below, DNAs are collected from mononuclear cells from adult T cell leukemia (ATL) patients, and the proviral HTLV-1 gene causative of ATL is detected by inverse PCR and subsequent Southern blot, thereby diagnosing ATL. A preferred example of the examination is the examination of nucleic acid by which such a pathogenic virus or a marker gene for various cancer cells is detected. Examples of such genes include HTLV-1 (ATL), Rb (retinoblastoma, lung cancer, breast cancer), p53 (colon cancer, breast cancer, lung cancer and the like), WTI (Wilms tumor), APC (colon cancer, gastric cancer), p1b (melanoma, esophagus cancer), NFI (melanoma, neuroblastoma), NF2 (meningioma, esophagus cancer), VHL (renal cancer), DPC-4 (pancreatic cancer), SMAD2 (colon cancer), PTEN (glioblastoma), PTC (dermal basal cell carcinoma), int-2/hst-1/cycD1 (head and neck cancer, esophagus cancer, bladder cancer), MDM-2 (sarcoma, brain tumor), erbBI (polymorphic glioma, breast cancer), erbB2(neu) (breast cancer, gastric cancer, ovarian cancer), c-myc (uterine cancer, small cell carcinoma of the lung, breast cancer), N-myc (neuroblastoma, small cell carcinoma of the lung, sarcoma), H-ras (uterine cancer), K-ras (gastric cancer), c-met (gastric cancer), K-sam (gastric cancer), AKT-1, AKT-2(S/T-PK) (both of them are markers of gastric cancer and ovarian cancer), and Aurora-2(S/T-PK) (colon cancer). Other than the examination of nucleic acids, examinations of EGF receptor (breast cancer and the like), p53 protein (colon cancer, liver cancer), vascular epidermal growth factor (VEGF) (liver cancer, colon cancer and the like), TGF-$\beta$, annexin'-I and the like are exemplified. It is also preferred to search the expression of a gene such as 4F2 gene or PCD1 gene by RT-PCR, of which expression is ubiquitously increased in cancer cells (see Example 4). The nucleotide sequences of these pathogenic virus genes and cancer marker genes or cDNAs thereof are known and are included in databases such as GenBank (freely available at http://www.ncbi.nlm.nih.gov/Genbank/index.html). Therefore, by carrying out a search using the name of the pathogenic virus or the cancer marker as the keyword, the nucleotide sequence thereof may easily be found. If the nucleotide sequence of the pathogenic virus gene or the cancer marker gene or the cDNA thereof is known, whether the cells contain the pathogenic virus gene or the cancer marker gene or not, or the gene is expressed in the cells or not may be determined by, for example, subjecting the gene or cDNA to a well-known nucleic acid-amplification method such as PCR for amplifying an optional region in the gene or cDNA, and determining whether amplification occurs or not.

Since the nucleic acid-amplification methods such as PCR and the methods for the subsequent detection of the amplification product are well-known in the art and since reagent kits and apparatuses therefor are commercially available, those skilled in the art can easily practice the nucleic amplification methods. The size of the primers used in PCR is not less than 15 bases, preferably about 18 to 50 bases. By subjecting the target pathogenic virus gene or cancer marker gene or its cDNA to a nucleic acid-amplification method using a pair of primers which are a part of the target pathogenic virus gene or cancer marker gene or its cDNA, and a part of the complementary chain thereof, respectively, and using the test nucleic acid as the template, the test nucleic acid is amplified. In contrast, if the test nucleic acid is not contained in the test sample, amplification of the nucleic acid does not occur. Therefore, by detecting the amplification product, whether the test nucleic acid exists in the sample or not can be determined. Detection of the amplification product may be carried out by a method in which the reaction solution after amplification is electrophoresed, and staining the bands with ethidium bromide or the like, or by a method comprising immobilizing the amplification product after electrophoresis on a solid phase such as a nylon membrane, hybridizing a labeled probe which specifically hybridizes with the test nucleic acid with the immobilized amplification product, and detecting the label after washing. The test nucleic acid in the sample may also be quantified by conducting the so called real-time detection PCR (see Examples below) using a quencher fluorescent pigment and a reporter fluorescent pigment. Since kits for real-time detection PCR are also commercially available, it can be easily carried out. Further, the test nucleic acid may also be semi-quantified based on the intensity of the electrophoretic band. Thc test nucleic acid may be a mRNA or a cDNA reverse-transcribed from the mRNA. In cases where a mRNA as a test nucleic acid is amplified, NASBA method (3SR method or TMA method) using the above-mentioned pair of primers may also be employed. Since NASBA method *per se* is well-known, and since the kits therefor are commercially available, NASBA may easily be carried out using the above-mentioned pair of primers.

The pathogenic virus gene or the cancer marker gene or the cDNA thereof may also be detected by a method using a nucleic acid probe. The method using a nucleic acid probe is also well-known, and if the nucleotide sequence of the test nucleic acid is known, the method may be carried out by hybridizing a labeled nucleic acid probe with the test nucleic acid, which probe has a nucleotide sequence complementary to a region of the test nucleic acid, and then detecting the label. As the probe, a nucleic acid having a nucleotide sequence complementary to a part of the nucleotide sequence of the pathogenic virus gene or cancer marker gene or cDNA thereof, which nucleic acid is labeled with a fluorescent label, radioactive label, biotin label or the like may be employed. Whether the test nucleic acid exists in the sample or not may be determined by immobilizing the test nucleic acid or an amplification product thereof on a solid phase, hybridizing it with the labeled probe, and measuring the label bound to the solid phase after washing. Alternatively, the detection of the test nucleic acid may also be carried out by immobilizing a nucleic acid for measurement on a solid phase, hybridizing the test nucleic acid therewith and detecting the test nucleic acid bound to the solid phase by a labeled probe or the like. In such a case, the nucleic acid for measurement immobilized on the solid phase is also called a probe.

As the pathological tests and biochemical tests of the cancer cells trapped on the magnetic beads by the above-described method, the following methods are exemplified. That is, examples of the pathological tests include detection of Philadelphia chromosome in chronic myelocytic leukemia, detection of signet-ring cell carcinoma observed in gastric cancer cells or the like, detection of coffee bean-like morphology observed in the nuclei of cancer cells, and detections of tumor markers such as CEA (carcinoembryonic antigen), AFP(alfa-feto protein), CA19-9, DUPAN-2, TPA (tissue polypeptide antigen) and the like. Examples of the biochemical tests include measurements of the above-mentioned tumor markers expressed by cancer cells by ELISA and measurements of isozymes (e.g., LDH isozyme and 5'-NPD-V isozyme) expressed by cancer.

The present invention will now be described more concretely by way of examples thereof. It should be noted that the present invention is not restricted to the examples below.

Reference Example 1 Detection of ATL

1. Preparation of Samples

Mononuclear cells were separated from peripheral blood from 7 acute ATL patients, 5 chronic ATL patients, 9 smoldering ATL patients, 42 healthy ATL carriers and 8 healthy individuals (not infected with HTLV-I), respectively. The separation of the mononuclear cells was carried out as follows. In a centrifugal tube, 5 ml of Lymphaprep (trademark) (Axis-Sheld PoC AS, Oslo, Norway) was placed and 5 ml of venous blood supplemented with heparin was gently overlaid thereon. Then the sample was centrifuged at 400 x g for 30 minutes, and the mononuclear cells suspended in the form of a band between the blood plasma and the separation solution were recovered with a capillary pipette. The recovered mononuclear cells was subjected to centrifugal washing three times with phosphate-buffered saline (PBS), and the resultant was used in the subsequent experiments.

2. Flow Cytometry

Mouse anti-SF-25 monoclonal antibody (produced by ATCC No. HB9599) was labeled with FITC by a conventional method. Using the fluorescence-labeled anti-SF-25 monoclonal antibody, flow cytometry was carried out for the mononuclear cells separated in 1 described above. The flow cytometry was carried out as follows. From a mononuclear cell suspension in PBS of which population density was adjusted to $5 \times 10^6$ cells/ml, 0.1 ml aliquots were sampled into two small test tubes. To one of the test tubes, 10 μl of diluted fluorescence-labeled SF-25 monoclonal antibody was added. To the other test tube, 10 μl of FITC-labeled mouse IgG1 was added. Each of the mixtures was allowed to react at 4°C for 30 minutes while gently agitating the mixture at 10 minutes' intervals. After the reaction, PBS was added, and centrifugal washing was carried out twice. Thereafter, the mixture was applied to a flow cytometer EPICS XL (trademark), Coulter, Miami, Florida, U.S.A.), and about 10,000 cells were counted using FITC-labeled mouse IgG1 as a control, to determine the ratio of the positive cells.

By the above-described flow cytometry, the ratio of the mononuclear cells expressing SF-25 antibody was determined. The results are shown in Table 1 below and in Fig. 1.

Table 1

| Examinee | Acute ATL Patients | Chronic ATL Patients | Smoldering ATL Patients | Healthy HTLV-I Carriers | Healthy Individuals |
|---|---|---|---|---|---|
| Ratio (%) of Mononuclear Cells Expressing SF-25 Antigen (average) | 42.7 | 27.9 | 15.2 | 0.6 | 0.4 |

As is apparent from Table 1 and Fig. 1, by using SF-25 antigen on mononuclear cells as a marker, for ATL, the group of healthy individuals + healthy carriers and the group of acute + chronic + smoldering may be clearly distinguished.

Example 1

Collection of SF-25-expressing Mononuclear Cells Using Magnetic Beads and Test of Nucleic Acids Thereof

Whether or not HTLV-1 gene was inserted in the form of provirus in the chromosomal DNAs in the mononuclear cells expressing SF-25 antigen was examined by inverse PCR and Southern blot. These were carried out as follows.

(1) Collection of Mononuclear Cells Expressing SF-25 Antigen

In 60 μl of phosphate-buffered saline (pH 7.2, containing 0.5% bovine serum albumin and 2 mM EDTA, hereinafter referred to as "buffer"), $10^7$ mononuclear cells separated from peripheral blood from each smoldering ATL patient were suspended. Then 10 μl of FITC-labeled mouse anti-SF-25 monoclonal antibody (produced by ATCC No. HB9599) solution (concentration: 0.1%) was added thereto and the mixture was allowed to react at 4°C for 5 minutes, followed by washing twice to remove the non-adsorbed antibody. To the resulting cells, 90 μl of buffer was added to suspend the cells again, and 10 μl of anti-FITC micromagnetic beads (Miltenyi Biotec GmbH, Bergish Gladbach, Germany, particle diameter: 50 nm) was added to the suspension, followed by allowing the labeling at 6°C for 15 minutes, thereby binding the mononuclear cells having SF-25 antigen on their surfaces to the magnetic beads. The cells were washed twice to remove the non-adsorbed beads, and the resulting cells were re-suspended in 500 μl of buffer. Using MACS Midi Set (Miltenyi Biotec GmbH, Bergish Gladbach, Germany), the cells were applied to a MACS Separation Positive Selection MS Column (Miltenyi Biotec GmbH, Bergish Gladbach, Germany) (washed once with 500 μl of buffer), and the cells labeled with the magnetic beads (SF-25 antigen-positive cells) and the cells not labeled with magnetic beads (SF-25 antigen-negative cells) were fractioned by magnetic force. The fractioned cells were used in the subsequent experiments as samples.

(2) Inverse PCR

Inverse PCR was performed as follows based on the report by Takemoto S et al (Blood Vol 84 No9 3080-3085, 1994). Chromosomal DNAs were extracted from each sample using DNAzol (Molecular Research Center, Inc., Montgomery Rd., Cincinnati, Ohio), and the DNAs were cleaved by *Sau* 3AI, followed by subjecting the resultant to self ligation using T4 DNA ligase. By this method, DNA consisting of HTLV-1 5'LTR and the gag sequence, and DNA consisting of HTLV-1 3'-LTR and chromosomal DNA are constructed. To remove the DNA consisting of the 5' proviral DNA of HTLV-1, the mixture was treated with *Sac* II under heat. Using the resulting DNA as a template, two-step nested PCR was performed. The first step PCR was performed using primer 1: 5'-aagccggcagtcagtcgtga-3' (8946-8927nt in nucleotide sequence of HTLV-1) and primer 2: 5'-aagtaccggcaactctgctg-3' (8958-8977nt in nucleotide sequence of HTLV-1). Then the second step PCR was performed using primer 3: 5'-gaaagggaaaggggtggaac-3' (8924-8905nt in nucleotide sequence of HTLV-1) and primer 4: 5'-ccagcgacagcccattctat-3' (8986-9005nt in nucleotide sequence of HTLV-1). Each PCR was performed using Thermal Cycler by repeating 50 times in the first step and 35 times in the second step the cycle of 94°C for 20 seconds, 55°C for 20 seconds and 72°C for 30 seconds. A 5 μl aliquot of the PCR product was sampled and subjected to electrophoresis on 2% agarose gel. The gel was stained with ethidium bromide and the band was examined for the existence of the incorporation of clonal HTLV-1.

(3) Southern Blot

The above-described electrophoresed product was transferred to a nylon membrane filter, and incorporation of HTLV-1 was examined using an oligonucleotide (5'-ctccaggagagaaatttagtacac-3', 9012-9035nt in nucleotide sequence of HTLV-1) as a probe. According to the report by Takcmoto et al., the U5 region of 3'LTR of HTLV-1 containing the chromosomal gene is thought to be amplified by this method. Although the incorporation of HTLV-1 gene in ATL patients is random between different cases, the incorporation of HTLV-1 gene in ATL cells in one patient is monoclonal, so that whether the amplification of the gene is monoclonal or not can be determined by amplifying the U5 region in the 3'LTR of HTLV-1 containing the chromosomal DNA. In fact, they confirmed it by sequencing the DNA in the U5 region of the 3'LTR of HTLV-1 containing the chromosomal DNA.

As a result, in the mononuclear cells expressing SF-25 antigen, the HTLV-I proviral DNA was monoclonally incorporated, and in the SF-25-negative cells, proviral HTLV-I was not detected. By this, it was confirmed that acute, chronic and smoldering ATL may be detected by the method of the present invention.

Example 2

Synthesis of Magnetic Beads Sensitized with Anti-SF-25 Antibody

Magnetic beads for separation of cells (DYNAL, M-450 Goat anti-Mouse IgG) in the form of a suspension in an amount of 10 mL (solid content: 300 mg) were rinsed 4 times with PBS, and then dispersed in 6 mL of PBS. To this dispersion, 0.60 mL of an anti-SF-25 antibody (1.0 mg/mL) was added, and the mixture was allowed to react at 20°C for 4 hours, followed by washing the resultant with physiological saline to obtain magnetic beads sensitized with anti-SF-25 antibody. The obtained beads were dispersed in phosphate buffer solution (PBS) supplemented with 0.1% bovine serum albumin to a solid content of 2%.

Example 3

Synthesis of Magnetic Beads Sensitized with Anti-SF-25 Antibody (Chemical Antibody-Sensitization Method)

In MES buffer solution (5 mM, pH 6.0), 100 mg of magnetic beads for separation of cells (DYNAL, M-270 Carboxylic Acid) were suspended to a solid content of 5%. To bind an antibody to the carboxyl groups on the magnetic beads, 20 mg of EDC hydrochloric acid salt (1-ethyl-3(3-dimethylaminopropyl)carbodiimide hydrochloride) which was a water-soluble carbodiimide reagent was added, and the mixture was allowed to react at 20°C for 1 hour to activate the beads. Then the beads were washed once with HEPES buffer solution (0.1 M, pH 7.4) and then dispersed in 2 mL of the same buffer solution. To the resulting dispersion, 0.4 mL of anti-SF-25 antibody (1.0 mL/ml) was added, and the resulting mixture was allowed to react at 20°C for 4 hours, followed by washing the resultant with physiological saline to obtain magnetic beads to which anti-SF-25 antibody was chemically bound. The obtained beads were dispersed in PBS supplemented with 0.1 % BSA to a solid content of 2%.

Example 4

Separation of KUT-2 Cells

A 5 mL of citrated blood sampled from a healthy donor was centrifuged and the buffy coat was collected. To the buffy coat, 10 mL of 0.17 M aqueous ammonium chloride solution was added and the mixture was left to stand at room temperature for 10 minutes, followed by centrifuging the mixture. The obtained precipitate was washed twice with PBS containing 2 mM EDTA, and suspended in 4.5 mL of PBS containing 0.5% BSA and 0.6% citric acid to obtain normal human nucleated cell suspension.

A cell culture of KUT-2 cells (Hanada S, Tsubai F and Namba Y. The Characteristics of T-cell lines derived from peripheral blood of patients with adult T-cell leukemia-lymphoma. Recent Advances in RES Research 1985; 25: 124-133) in RPMI1640 medium supplemented with 10% FCS, cultured at 37°C in an incubator under 5% $CO_2$ was centrifuged. The obtained precipitate was washed twice with PBS containing 2 mM EDTA, and suspended in 4.0 mL of PBS containing 0.5% BSA and 0.6% citric acid to obtain KUT-2 cell suspension having a population density of 6.0 x $10^5$ cells/mL. The normal human cell suspension and the KUT-2 cell suspension obtained above were mixed so as to attain the number of fed cells shown in Table 5, and the mixture was diluted with PBS containing 0.5% BSA and 0.6% citric acid to a total volume of 1.0 mL. Selective separation of KUT-2 cells from the cell mixture was tried using the magnetic beads sensitized with the anti-SF-25 antibody, prepared in Examples 2 and 3. To 1.0 mL of the cell mixture solution, 50 μL (beads content: 1 mg) of the magnetic beads sensitized with the anti-SF-25 antibody prepared in Example 2 or 3 was added, and the resulting mixture was mixed by inversion for 30 minutes at 4°C. The magnetic beads were separated by magnetic separation, and the beads were washed three times with PBS containing 0.5% BSA and 0.6% citric acid, thereby removing the cells adsorbed to the beads by weak interaction. The beads were then washed once with PBS containing 2 mM EDTA, and separated by magnetic force, followed by removing the washing solution to eliminate citric acid which adversely affects the nucleic acid-amplification reaction. To elute nucleic acids from the cells trapped on the magnetic beads, 50 μL of Proteinase K solution (0.8 mg/mL) diluted with 10 mM Tris-HCl (pH8.3) was added and the mixture was allowed to react at 55°C for 15 minutes to lyse the cells. Then the reaction was allowed to occur at 95°C for 20 minutes to inactivate Proteinase K used for the cell lysis and to inactivate the substances originated from the cells, which inhibit the nucleic acid-amplification reaction.

Using 20 μL aliquot of the nucleic acid solution, quantitative PCR was carried out to count the number of the KUT-2 cells and the number of the normal human cells trapped on the magnetic beads. The quantification of the KUT-2 cells was carried out by amplifying and quantifying a 102mer region in the pX gene region originated from HTLV-1 virus. The nucleotide sequences of the primers and of the probe are shown in Table 2.

The normal human cells were counted by amplifying and quantifying a 110mer region in β-globin gene to determine the total number of the captured KUT-2 cells and normal human cells, and then subtracting the number of the KUT-2 cells. The nucleotide sequences of the primers and of the probe are shown in Table 3.

The quantitative PCR was performed using ABI PRISM 7700 Sequence Detection System (Applied Biosystems). The number of the cells was determined based on the number of cycles (Th cycle) at which the fluorescence intensity exceeded a threshold value (Th), by extrapolating a calibration curve prepared at the same time.

For the preparation of calibration curves of the pX gene and the β-globin gene, a serial dilution of nucleic acid solution was used, which was obtained by adding Proteinase K solution (0.8 mg/mL) to a prescribed amount of KUT-2 cells or normal human cells, allowing the mixture to react at 55°C for 15 minutes to lyse the cells and then reacting the mixture at 95°C for 20 minutes. The relationship between the number of cycles and the fluorescence intensity when the pX gene was amplified is shown in Fig. 2. Quantitative amplification was confirmed between 0.5 cell to $5 \times 10^5$ cells. At this time, (Number of Cells) = $10^{\wedge}(30.77/3.46\text{-Th cycle}/3.46)$, correlation coefficient r = 1.00.

Table 2

| Quantification of pX Gene | 5'-sequence-3' |
|---|---|
| Forward Primer | TTC CCA GGG TTT GGA CAG AG |
| Reverse Primer | CGA AGA TAG TCC CCC AGA GA |
| TaqMan Probe | FAM-ATA CCC AGT CTA CGT GTT TGG AGA C-TAMRA |

Table 3

| Quantification of β-globin Gene | 5'-sequence-3' |
|---|---|
| Forward Primer | ACA CAA CTG TGT TCA CTA GC |
| Reverse Primer | CAA CTT CAT CCA CGT TCA CC |
| TaqMan Probe | FAM-AAC AGA CAC CAT GGT GCA TCT GAC T-TAMRA |

The PCR solution contained reagents for hot-start (Roche Diagnostics) and had the composition shown in Table 4. To 30 μL of the solution, 20 μL of the nucleic acid extract obtained above was added, and the resulting mixture was subjected to amplification.

Table 4

| Composition of PCR Solution | |
|---|---|
| PCR grade distilled water | 17.95 μl |

Table 4 (continued)

| Composition of PCR Solution | |
|---|---|
| x 10 reaction buffer | 3 µl |
| MgCl$_2$ (25mM) | 5 µl |
| dATP, dGTP, dCTP (10mM) | each 0.5 µl |
| dUTP (20mM) | 0.5 µl |
| forward primer (50 µM) | 0.6 µl |
| reverse primer (50 µM) | 0.6 µl |
| TaqMan probe (25 µM) | 0.2 µl |
| Fast Start DNA polymerase (5U/µL) | 0.4 µl |
| ROX reference dye (Invitrogen) | 0.25 µl |
| | Total 30 µl |

The PCR was performed as follows:

| Activation of Polymerase | 95°C for 10 minutes |
|---|---|
| PCR Cycle | 60°C for 60 seconds |
| | 95°C for 10 seconds |

This PCR cycle was repeated 50 times.

From the determined number of KUT-2 cells and of normal human cells, the ratio of capturing KUT-2 cells and purity thereof were calculated according to the following equation, and the measured values of 10 runs were averaged, which are shown in Table 5.

$$\text{Ratio of Capturing (\%) = Number of Captured KUT-2 Cells/Number of Fed KUT-2 Cells}$$

$$\times\ 100$$

$$\text{Purity (\%) = Number of Captured KUT-2 Cells/(Number of Captured KUT-2 Cells +}$$

$$\text{Number of Captured Normal Human Cells)} \times 100$$

Table 5

| Beads Used | Number of Fed KUT-2 CELLS | Number of Fed Human Normal Cells | Number of Captured KUT-2 Cells | Number of Captured Human Normal Cells | Ratio of Captured KUT-2 Cells (%) | Purity of KUT-2 Cells (%) |
|---|---|---|---|---|---|---|
| Example 2 | 3.0 x 10$^5$ | 6.0 x 10$^5$ | 2.8 x 10$^5$ | 2.7 x 10$^3$ | 92 | 99 |
| Example 2 | 3.0 x 10$^4$ | 6.0 x 10$^4$ | 2.9 x 10$^4$ | 1.2 x 10$^3$ | 95 | 96 |
| Example 2 | 3.0 x 10$^3$ | 6.0 x 10$^3$ | 2.7 x 10$^3$ | 2.6 x 10$^2$ | 90 | 91 |
| Example 2 | 3.0 x 10$^2$ | 6.0 x 10$^2$ | 2.8 x 10$^2$ | 1.2 x 10$^1$ | 94 | 96 |
| Example 3 | 3.0 x 10$^5$ | 6.0 x 10$^5$ | 2.9 x 10$^5$ | 2.7 x 10$^3$ | 98 | 99 |
| Example 3 | 3.0 x 10$^4$ | 6.0 x 10$^4$ | 2.9 x 10$^4$ | 9.0 x 10$^2$ | 97 | 97 |
| Example 3 | 3.0 x 10$^3$ | 6.0 x 10$^3$ | 3.0 x 10$^3$ | 6.3 x 10$^2$ | 99 | 98 |
| Example 3 | 3.0 x 10$^2$ | 6.0 x 10$^2$ | 3.0 x 10$^2$ | 1.3 x 10$^1$ | 99 | 96 |

As shown in Table 5, by using the magnetic beads sensitized with the anti-SF-25 antibody, the target KUT-2 cells were able to be recovered with a high efficiency and high purity by a simple operation. The DNA eluted from the recovered cells had a sufficient purity for supplying to quantitative PCR. By the method of the present invention, the accuracy of gene diagnosis can be increased, and diseases such as cancers can be detected at an earlier stage.

Example 4

Extraction of Nucleic Acid from Human Cancer Cells and Amplification

Heparinated blood collected from a healthy donor was centrifuged and buffy coat was recovered. The buffy coat was overlaid on Ficoll-Paque Plus (Amersham Pharmacia) and the resultant was centrifuged. The resultant was suspended in PBS and the human normal nucleated cell fraction was recovered.

Human gastric cancer cells OCUM-2M LN (Fujihara T, Sawada T, Chung K H-YS, Yashiro M, Inoue T and Sowa M. Establishment of lymph node metastatic model for human gastric cancer in nude mice and analysis of factors associated with metastasis), lung cancer cells Calu-6 (ATCC Number: HTB-56, J Fogh (editor). Human tumor cells in vitro. New York: Plenum Press; 1975. pp.115-159.), pancreatic cancer cells Capan-2(ATCC Number: HTB-80, Dahiya R, Kwak KS, Byrd JC, Ho S, Yoon W, and Kim YS. Mucin synthesis and secretion in various human epithelial cancer cell lines that express the MUC-1 mucin gene. Cancer Research 1993; 53: 1437-1443.), colon cancer cells HT-29 (ATCC Number: HTB-38, J Fogh (editor). Human tumor cells in vitro. pp.115-159. New York: Plenum Press; 1975.), uterine cancer cells HeLa (ATCC Number: CCL-2 Gey GO, Coffman WD and Kubicek MT. Tissue culture studies of the proliferative capacity of cervical carcinoma and normal epithelium. Cancer Research 1952; 12: 264-265.) were suspended in PBS respectively, to prepare cell suspensions having a prescribed concentration.

To extract nucleic acids from human normal cells and various human cancer cells, SMITEST EX-R&D (Genome Science Laboratories) was used. More specifically, after centrifuging the cell suspension, supernatant was removed, and 15 µl of enzyme solution, 480 µl of sample diluent and 5 µl of coprecipitation agent were added thereto, followed by allowing the resulting mixture at 55°C for 30 minutes after mixing. To the resultant, 400 µl of protein solution was added and the resulting mixture was allowed to react at 55°C for 15 minutes after mixing. Then 800 µl of isopropanol was added thereto, and the mixture was centrifuged after mixing, followed by removal of the supernatant. To the resultant, 500 µl of 70% ethanol was added, and the resultant was centrifuged after washing, followed by removal of the supernatant similarly. The resultant was dissolved in DNase/RNase free water to obtain a nucleic acid extract solution.

From this nucleic acid extract solution, genes were amplified and quantified by quantitative RT-PCR.

For the reverse transcription reaction, Omniscript reverse transcriptase (QIAGEN) was used, and the composition shown in Table 6 was employed. To 15 µl aliquot thereof, 5 µl of the nucleic acid extract obtained above was added and the reaction was allowed to occur.

Table 6

| Composition of Reverse Transcription Reaction Solution | |
|---|---|
| x 10 Reaction buffer | 2 µl |
| dNTP mix (5mM) | 2 µl |
| Random hexamer primer (CLONTECH) (20 µM) | 1 µl |
| RNasin ribonuclease inhibitor (Promega) (40 Units/µl) | 0.25 µl |
| Omniscript reverse transcriptase | 1 µl |
| RNase-free water | 8.75 µl |
| Total | 15 µl |

The reverse transcription reaction was carried out under the following conditions:

| Activation of reverse transcriptase | 37°C for 60 minutes |
|---|---|
| Inactivation of reverse transcriptase | 93°C for 5 minutes |

Then the obtained single-stranded DNA was amplified and quantified by PCR. As the target genes to be amplified and quantified, a 101 mer region in PCD1 gene and a 101mer region in 4F2 gene were employed, of which expressions are increased in various cancer cells. The nucleotide sequences of the primers and the probes are shown in Tables 7 and 8. On the other hand, human endogenous housekeeping genes, that is, genes of human β-actin, human GAPDH and 18S ribosomal RNA were amplified and quantified similarly.

Table 7

| Quantification of PCD1Gene | 5'-sequence-3' |
|---|---|
| Forward primer | GAC AAG GCT GCC CTC TCC TA |
| Reverse primer | TTA AAT CAA GAC CAG ATG TGG AAG AC |
| TaqMan probe | FAM-CTT TCC CAA GAC CAG GCT GCC ACT TCT-TAMRA |

Table 8

| Quantification of 4F2 Gene | 5'-sequence-3' |
|---|---|
| Forward primer | TCC TTC TTG CCG GCT CAA C |
| Reverse primer | GCA TCC AGG CCA ATC TCA TC |
| TaqMan probe | FAM-CGA CTC TAC CAG CTG ATG CTC TTC ACC C-TAMRA |

The quantitative PCR was performed using ABI PRISM 7700 Sequence Detection System (Applied Biosystems). The PCR solution contained QuantiTect Probe PCR (QIAGEN), and had the composition shown in Table 9. To 47 µl aliquot thereof, 6 µl of the reaction solution obtained in the reverse transcription reaction was added, and the mixture was subjected to amplification.

Table 9

| Example of Composition of Quantitative PCR Solution | |
|---|---|
| 2 x QuantiTect Probe PCR Master Mix | 25 µl |
| Forward primer (100 µM) | 0.2 µl |
| Reverse primer (100 µM) | 0.2 µl |
| TaqMan probe (13 µM) | 0.36 µl |
| Distilled water | 18.24 µl |
| Total | 44 µl |

The PCR was carried out under the following conditions:

| Activation of polymerase | 95°C for 15 minutes |
|---|---|
| PCR cycle | 94°C for 15 seconds |
| | 60°C for 60 seconds |

This PCR cycle was repeated 50 times. The quantification of the expression of each gene was determined based on the threshold value (Th), by extrapolating a calibration curve prepared at the same time. For the preparation of calibration curves, a serial dilution of a prescribed amount of cells was used.

For the human normal cells and for the human cancer cells, the expression ratios of the PCD1 gene and 4F2 gene, respectively, to that of the human endogenous housekeeping genes were determined. It was proved that expressions of 4F2 gene and PCD1 gene were higher than in human normal cells from the level of several cells.

Amplifications of the genes which are strongly expressed in cancer cells were observed in a small amount of cells too. By using the magnetic beads sensitized with anti-cancer-specific SF-25 antibody, cancer cells in the blood may be recovered at a high efficiency to a high purity, and the cancer cells may be detected by the quantitative RT-PCR of a specific gene. By the method of the present invention, early detection and diagnosis of cancers using a sample separated from the body, such as blood, may be attained.

Industrial Availability

By the method of the present invention, cancer cells can be examined simply and efficiently without using an expensive apparatus such as a cell sorter.

**Claims**

1. A method for examining cancer cells, comprising binding cancer cells separated from the body, which cells express SF-25 antigen on their surfaces, to magnetic beads utilizing antigen-antibody reaction between said cancer cells and an anti-SF-25 antibody or antigen-binding fragment thereof, then collecting said magnetic beads by magnetic force, and examining said cancer cells bound to said magnetic beads.

2. The method according to claim 1, wherein the step of binding said cancer cells to said magnetic beads is carried out by subjecting magnetic beads on which said anti-SF-25 antibody or antigen-binding fragment thereof is immobilized and said cancer cells to antigen-antibody reaction, or by subjecting a labeled or non-labeled anti-SF-25 antibody or antigen-binding fragment thereof and said cancer cells to antigen-antibody reaction, and subsequently thereto or simultaneously therewith, reacting magnetic beads on which a substance that specifically binds to the generated antigen-antibody complex is immobilized with the generated antigen-antibody complex.

3. The method according to claim 1 or 2, wherein said cancer cells are those contained in blood, cerebrospinal fluid, bone marrow, pleural effusion, ascites, pancreatic juice, duodenal juice, bile, feces or urine.

4. The method according to claim 4, wherein said cancer cells are leukemia cells, colon cancer cells, small intestinal cancer cells, gastric cancer cells, esophagus cancer cells, bile duct cancer cells, gallbladder cancer cells, thyroid cancer cells, parathyroid cancer cells, prostate cancer cells, uterine cancer cells, ovarian cancer cells, choriocarcinoma cells, orchioncus cells, bladder cancer cells, renal cancer cells, adrenal cancer cells, brain tumor cells, melanoma cells, skin cancer cells, lung cancer cells, breast cancer cells, pancreatic cancer cells or liver cancer cells.

5. The method according to claim 4, wherein said cancer cells are leukemia cells, human gastric cancer cells, lung cancer cells, pancreatic cancer cells, colon cancer cells or uterine cancer cells.

6. The method according to claim 5, wherein said cancer cells are leukemic mononuclear cells.

7. The method according to any one of claims 1 to 6, wherein the examination is an examination of nucleic acids.

8. A reagent for examination of cancer cells for carrying out said method according to any one of claims 1 to 7, comprising magnetic beads on which an anti-SF-25 antibody or antigen-binding fragment thereof is immobilized.

9. Use of the magnetic beads on which an anti-SF-25 antibody or antigen-binding fragment thereof is immobilized for the production of a reagent for examination of cancer cells.

SF-25-positive Cells in Peripheral Blood Mononuclear Cells in Each Clinical Form

Fig. 1

Fig. 2

### INTERNATIONAL SEARCH REPORT

| International application No. |
|---|
| PCT/JP03/14201 |

A. CLASSIFICATION OF SUBJECT MATTER
   Int.Cl⁷ G01N33/547, G01N33/543, G01N33/553

According to International Patent Classification (IPC) or to both national classification and IPC

B. FIELDS SEARCHED

Minimum documentation searched (classification system followed by classification symbols)
   Int.Cl⁷ G01N33/53-579

Documentation searched other than minimum documentation to the extent that such documents are included in the fields searched
   Jitsuyo Shinan Koho          1922-1996    Toroku Jitsuyo Shinan Koho    1994-2003
   Kokai Jitsuyo Shinan Koho    1971-2003    Jitsuyo Shinan Toroku Koho    1996-2003

Electronic data base consulted during the international search (name of data base and, where practicable, search terms used)
   BIOSIS(DIALOG), WPI(DIALOG)

C. DOCUMENTS CONSIDERED TO BE RELEVANT

| Category* | Citation of document, with indication, where appropriate, of the relevant passages | Relevant to claim No. |
|---|---|---|
| Y | SUZUKI et al., "Chimeric SF-25 monoclonal antibody mediated apoptosis of adult T-cell leukemia cells by human lymphocytes", Blood Vol.90 (10 SUPPL. 1 PART 1), page 513A, 15 November, 1997 (15.11.97) | 1-9 |
| Y | JP 9-510779 A (Øystein FODSTAD), 28 October, 1997 (28.10.97), Claims & US 6265229 A          & WO 95/24648 A & EP 749580 A | 1-9 |

☐ Further documents are listed in the continuation of Box C.       ☐ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier document but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 28 November, 2003 (28.11.03) | 09 December, 2003 (09.12.03) |

| Name and mailing address of the ISA/ | Authorized officer |
|---|---|
| Japanese Patent Office | |
| Facsimile No. | Telephone No. |

Form PCT/ISA/210 (second sheet) (July 1998)

SEQUENCE LISTING

<110> TAKAHASHI Hiroshi and HANADA Shuichi

<120> Method for Examining Cancer Cells and Reagent Therefor

<130> 03PF273-PCT

<160> 17

<210> 1

<211> 20

<212> DNA

<213> Artificial Sequence

<220>

<223> Oligonucleotide forward primer used in inverse PCR for amplifying a region of HTLV-1 gene

<400> 1

aagccggcag tcagtcgtga                                              20

<210> 2

<211> 20

<212> DNA

<213> Artificial Sequence

<220>

<223> Oligonucleotide reverse primer used in inverse PCR for amplifying a region of HTLV-1 gene

<400> 2

aagtaccggc aactctgctg                                              20

<210> 3

<211> 20

<212> DNA

<213> Artificial Sequence

<220>

<223> Oligonucleotide forward primer used in inverse PCR for amplifying a region of HTLV-1 gene

<400> 3

gaaagggaaa ggggtggaac                                                      20


<210> 4

<211> 20

<212> DNA

<213> Artificial Sequence

<220>

<223> Oligonucleotide reverse primer used in inverse PCR for amplifying a region of HTLV-1 gene

<400> 4

ccagcgacag cccattctat                                                      20


<210> 5

<211> 24

<212> DNA

<213> Artificial Sequence

<220>

<223> Oligonucleotide probe used for detecting a region of HTLV-1 gene

<400> 5

ctccaggaga gaaatttagt acac                                                 24


<210> 6

<211> 20

<212> DNA

<213> Artificial Sequence

<220>

<223> Oligonucleotide forward primer used for measuring pX gene in HTLV-1 gene

<400> 6

ttcccagggt ttggacagag                                                    20


<210> 7

<211> 20

<212> DNA

<213> Artificial Sequence

<220>

<223> Oligonucleotide reverse primer used for measuring pX gene in HTLV-1 gene

<400> 7

cgaagatagt cccccagaga                                                    20


<210> 8

<211> 25

<212> DNA

<213> Artificial Sequence

<220>

<223> Oligonucleotide TaqMan probe used for measuring pX gene in HTLV-1 gene

<400> 8

atacccagtc tacgtgtttg gagac                                              25

<210> 9

<211> 20

<212> DNA

<213> Artificial Sequence

<220>

<223> Oligonucleotide forward primer used for measuring beta-globin gen

e

<400> 9

acacaactgt gttcactagc                                                    20


<210> 10

<211> 20

<212> DNA

<213> Artificial Sequence

<220>

<223> Oligonucleotide reverse primer used for measuring beta-globin gen

e

<400> 10

caacttcatc cacgttcacc                                                    20


<210> 11

<211> 25

<212> DNA

<213> Artificial Sequence

<220>

<223> Oligonucleotide probe used for measuring beta-globin gene

<400> 11

aacagacacc atggtgcatc tgact          25


<210>  12

<211>  20

<212>  DNA

<213>  Artificial Sequence

<220>

<223>  Oligonucleotide forward primer used for measuring PCD1 gene

<400>  12

gacaaggctg ccctctccta          20


<210>  13

<211>  26

<212>  DNA

<213>  Artificial Sequence

<220>

<223>  Oligonucleotide reverse primer used for measuring PCD1 gene

<400>  13

ttaaatcaag accagatgtg gaagac          26


<210>  14

<211>  27

<212>  DNA

<213>  Artificial Sequence

<220>

<223>  Oligonucleotide TaqMan probe used for measuring PCD1 gene

<400>  14

ctttcccaag accaggctgc cacttct          27

<210> 15

<211> 19

<212> DNA

<213> Artificial Sequence

<220>

<223> Oligonucleotide forward primer used for measuring 4F2 gene

<400> 15

tccttcttgc cggctcaac                    19


<210> 16

<211> 20

<212> DNA

<213> Artificial Sequence

<220>

<223> Oligonucleotide reverse primer used for measuring 4F2 gene

<400> 16

gcatccaggc caatctcatc                    20


<210> 17

<211> 28

<212> DNA

<213> Artificial Sequence

<220>

<223> Oligonucleotide TaqMan probe used for measuring 4F2 gene

<400> 17

cgactctacc agctgatgct cttcaccc                    28